# EUROPEAN PATENT SPECIFICATION

(11) **EP 2 821 061 B1**
(45) Date of publication and mention of the grant of the patent: **20.12.2017**
(21) Application number: 14174889.7
(22) Date of filing: 30.06.2014
(51) Int. Cl.: A61K 9/00, A61K 9/16, A61K 31/00

(54) **NOVEL INHALATION FORMULATIONS**
NEUE PRÄPARATE FUR ATEMWEGE
NOUVELLE FORMULATION POUR INHALATION

(30) Priority: 01.07.2013 TR 201307891
(43) Date of publication of application: 07.01.2015
(73) Proprietor: Arven Ilac Sanayi Ve Ticaret A.S., Istanbul 34460 (TR)
(72) Inventor: Türkyilmaz, Ali, 34460 Istanbul (TR); Celik, Devrim, 34460 Istanbul (TR); Mutlu, Onur, 34460 Istanbul (TR)
(74) Representative: Sevinç, Erkan

(56) References cited:
- WO-A1-02/28368
- US-A1- 2003 180 227
- US-A1- 2004 062 719
- US-A1- 2004 258 626
- US-B1- 6 645 466

## Description

### Field of Invention

The present invention relates to a formulation for use via inhalation to prevent, treat, or alleviate the symptoms of respiratory diseases, particularly asthma and chronic obstructive pulmonary disease, as well as to a process for producing this formulation.

### Background of Invention

The respiratory tract comprises mainly the lungs and the entirety of the ducts and sacs from the mouth and nose to the alveoli. The respiratory tract has a direct functional relation with the muscle and skeletal system (primarily the diaphragm muscle) providing mechanical ventilation and the cardiovascular system providing blood circulation within the respiratory tract. There are many factors leading to respiratory diseases. These factors causing respiratory tract diseases (e.g. chronic obstructive pulmonary disease, lung cancer, cystic fibrosis, asthma, pneumonia, tuberculosis) include genetic factors, age, gender, race, infections, as well as environmental factors such as smoking or exposure to cigarette smoke, air pollution, seasonal factors, geographical conditions, professional factors, etc..

The drugs used in the treatment of respiratory diseases are administered through various routes (e.g. inhalation, oral, parenteral). However, the preferred route of administration of these drugs is inhalation, since they are directly delivered to the affected sites (airways) in high doses via this route, have a short onset time, and they lack or have minimal systemic side effects. Therefore, mainly the inhalation devices, metered dose inhalers, nebulizers, and dry powder inhalers have a widespread use.

Among the most frequently encountered problems in the use of metered dose inhalers are the lack of achieving an adequate "hand-inhalation" synchronization in terms of inhaler users, the decline in cognitive functions due to advanced age and mental disorders, and the inability of the users in recognizing the finished state of the inhaler drug due to the lack of a counter. As compared to other inhalation devices, nebulizers are difficult to carry and are expensive. Dry powder inhalers, in turn, are among the most preferred inhalation devices based on some advantages thereof in that they are used conveniently, are environmentally friendly due to the lack of propellants, are carried easily, etc.

The performance of a dry powder inhaler also depends on the formulation delivered to the lungs using this device besides the inhaler characteristics. Formulations which are delivered via dry powder inhalers are in dry powder form and have an improved stability, an easier use and a more efficient drug delivery as compared to those formulations delivered using nebulizers and metered dose inhalers.

In inhalation therapy, the size of the drug particles in a formulation administered via dry powder inhalers is closely related to the influence of the respective drug on the respiratory disease. In order to gain a therapeutic benefit from the inhaled drug particles, they have to be absorbed in the lungs, i.e. at the bronchial and alveolar sites. For this reason, dry powder formulations prepared for inhalation therapy are formulated using drug particles of a micronized size. The particle size has to be below 5 µm for the absorption of the drug particles to occur at the target sites. Particles of this size are also known as "inhalable particles". Since drug particles of a size above 5 µm are mostly accumulated in the oropharyngeal cavity, the target sites cannot be reached and therefore the desired therapeutic effect cannot be obtained.

Since the cohesion forces between micronized particles are too high, they tend to agglomerate and thus have weak flow properties. In order to develop a successful product and achieve an efficient inhalation, it is quite important to adjust and control the flow properties of the particles in the formulation. Mixing the cohesive particles during production is very problematic, and additionally, it is necessary to fill the dry powder formulations prepared into the blister cavities, capsules or reservoirs used in dry powder inhalers under high speed and with high dose accuracy. Further, the flowability of dry powder formulations also affects the drug performance by influencing some factors such as discharging a dose from the inhaler and aerosolizing the particles into inhalable particles during inhalation.

Having said that, the drugs used in inhalation therapy have a quite strong effect and the dose of a drug to be delivered to achieve a therapeutic benefit per inhalation is in the unit of micrograms. For this reason, it is quite difficult to portion a drug into the capsules, blister cavities or reservoirs under high accuracy and precision. Therefore, drug particles used in inhalation therapy are diluted using suitable carrier particles. Since the amount of carrier particles used for diluting the micronized drug particles is substantially high, the type of these carrier particles and the particle size distribution thereof significantly influences the properties such as flowability of the dry powder formulation.

A widely employed method to improve the flow properties of a dry powder formulation used for inhalation therapy involves subjecting the micronized drug particles together with the carrier particles to a spheroidizing process. However, using the spheroidizing process may not always be advantageous in improving the flow properties of the dry powder formulation, since the matters such as the active agent(s) and/or excipient(s) used therein may not always be suitable for spheroidizing, use of various additional equipments may be required for the spheroidizing process, the stability of the active agent(s) may be influenced negatively.
WO98/031352A1 discloses a dry powder composition comprising one or more pharmaceutically active substances and a carrier substance, wherein these substances are in a finely divided form and the poured bulk density of the formulation is between 0.28 g/ml and 0.38 g/ml. According to that document, the active substance(s) and the carrier substance in the dry powder composition is subjected to a micronisation process and turned into finely divided particles, and then converted into agglomerates using a spheroidizing process. Since the particle size of the finely divided particles is quite low, adhesion and cohesion forces between the particles are very high, and thus they are inclined to agglomerate. However, this is not advantageous in terms of ensuring the formulation's flowability and high dose accuracy. At the same time, finely divided particles having an inclination to agglomerate may lead to various problems during production and particularly during a mixing process, and it may be necessary to repeat the micronisation process to achieve content uniformity.
WO 98/031350A1 and WO 98/031351 A1 disclose dry powder compositions comprising budesonide and formoterol respectively having a poured bulk density between 0.28 g/ml and 0.38 g/ml. The same drawbacks given in the paragraph above are present here as well.

Document US2003180227 discloses a dry powder for inhalation purposes comprising micronized formoterol or Budesonide and lactose monohydrate as an inactive carrier in two different sizes such as >100 µm and <15 µm. Considering the foregoing, it would be quite advantageous to provide a dry powder formulation suitable for use in the treatment of respiratory diseases via inhalation, as well as having a good flowability and ensuring high dose accuracy for an active agent to be administered.

### Description of Invention

The present invention relates to a dry powder formulation suitable for use in dry powder inhalers, eliminating all aforesaid problems and bringing additional advantages to the relevant prior art.

The main object of the present invention is to provide a dry powder formulation with improved flow properties for use in the prevention, treatment, or in the alleviation of the symptoms of respiratory diseases, particularly asthma and chronic obstructive pulmonary disease. The dry powder formulation of present invention comprises: a. a micronized formoterol fumarate dihydrate and micronized budesonide and b. lactose monohydrate as a carrier comprising fine lactose monohydrate particles and coarse lactose monohydrate particles; d_{0.5} particle size by volume of the fine lactose monohydrate particles is between 0.5 µm and 25 µm and d_{0.5} particle size by volume of the coarse lactose monohydrate particles is between 40 µm and 200 µm characterized in that the poured bulk density of the dry powder formulation is between 0.42 g/ml and 0.57 g/ml. Another object of the present invention is to provide a dry powder formulation which is filled into blisters, capsules, and reservoirs suitably used in dry powder inhalers with high dose accuracy.

Another object of the present invention is to provide a dry powder formulation which guarantees to discharge a dose present in a blister, capsule or reservoir from the inhaler device upon inhalation and to deliver the drug particles to the target site at an inhalable particle size.

Another object of the present invention is to provide a dry powder formulation which has a good flowability and provides a high dosing accuracy, as well as ensures a good performance in terms of an emitted dose and the fine particle fraction (inhalable fraction) of the drug particles.

Another object of the present invention is to provide a method for preparing a dry powder formulation having the features fulfilling the objects stated above.

A novel dry powder formulation suitable for use in a dry powder inhaler has been developed to carry out all objects, referred to above and to be disclosed in the following detailed description.

According to an embodiment of the present invention, the dry powder formulation comprises formoterol fumarate dihydrate and budesonide and a carrier agent namely lactose monohydrate, of a micronized size.

According to another embodiment of the present invention, the dry powder formulation comprising fine carrier particles and coarse carrier particles, each are having a different d_{0.5} particle size by volume, the fine lactose monohydrate particles being between 0.5 µm and 25 µm and the coarse lactose monohydrate particles being between 40 µm and 200 µm. According to another embodiment of the present invention, the carriers provided in the dry powder formulation are identical and are lactose monohydrate. According to another embodiment of the present invention, the poured bulk density of the dry powder formulation comprising a micronized active agent and the carrier comprising fine carrier particles and coarse carrier particles is between 0.42 g/ml and 0.57 g/ml. According to another embodiment of the present invention, the d_{0.5} particle size by volume of the fine carrier particles of the dry powder formulation is between 0.5 µm and 25 µm, whereas the d_{0.5} particle size by volume of the coarse carrier particles of the dry powder formulation is between 40 µm and 200 µm.

According to the present invention, the d_{0.9} particle size by volume of the fine carrier particles in the dry powder formulation is lower than the d_{0.5} particle size by volume of the coarse carrier particles therein. Here, the d_{0.9} particle size by volume of the fine carrier particles is below 60 µm, preferably between 1 µm and 50 µm, and more preferably between 5 µm and 48 µm.

Additionally, the weight ratio of the fine carrier particles to the coarse carrier particles is between 1:99 and 50:50, preferably between 1:99 and 45:55, and more preferably between 1:99 and 30:70 in a dry powder formulation according to the present invention.

### Detailed Description of Invention

In order to gain benefit from drug particles used in inhalation therapy, the drug particles should be delivered to the lungs in an inhalable size. Since drug particles of an inhalable size are quite cohesive, they tend to agglomerate and have poor flow properties. Because of these features they possessed, they are produced and filled into inhaler blisters, capsules or reservoirs with difficulty. Therefore, micronized drug particles are diluted using carrier agents to improve the flow properties of the drug particles and to uniformly fill the drug particles into blisters, capsules, or reservoirs. Additionally, since the amount of the carrier particles are substantially higher than that of the drug particles, the properties of dry powder formulations are greatly influenced by the properties of the carrier particles.

Accordingly, the present invention provides a dry powder formulation comprising formoterol fumarate dihydrate and budesonide particles and lactose monohydrate particles suitable for use in dry powder inhalers for the treatment of respiratory diseases.

The flow properties of the dry powder formulation according to the present invention comprising micronized drug particles and carrier particles was surprisingly found to have a signification improvement when the formulation was formulated to have a poured bulk density between 0.42 g/ml and 0.57 g/ml. By improving the flowability of said dry powder formulation formulated to have a poured bulk density in the range indicated above, the formulation can be uniformly portioned into blisters, capsules or reservoirs suitably used in dry powder inhalers, and thus, any dose inhaled by a patient from the respective blister, capsule, or reservoir during inhalation can be delivered with a high dose accuracy. Having said that, the dry powder formulation with good flow properties contributes to an almost complete discharge of the powder from the inhaler during inhalation.

The poured bulk density of the dry powder formulation according to the present invention is between 0.42 g/ml and 0.57 g/ml.

The poured bulk density according to the present invention is measured using a known method as described in "Powder testing guide: Methods of measuring the physical properties of Bulk powders," L.Svarovsky, Elsevier Applied Science 1987, pp. 84-86. In general terms, this method is based on calculating the poured bulk density of a previously weighed powder by passing it through a funnel into a graduated container. After all of the powder is poured into the container, the amount of powder is divided by the volume of the container, and thus the poured bulk density is obtained.

In order to enable the carrier particles, which are used for improving the flow properties of the drug particles in the dry powder formulation for inhalation therapy, to carry the drug particles until the inhalation time, an adequate interaction should be present between the drug particles and the carrier particles. This interaction, however, should both be strong enough to prevent the separation of the drug particles from the carrier agent during production, transport and storage, and weak enough to let the drug particles leave the carrier particles in an inhalable size to be delivered to the lungs upon inhalation. For the drug particles to provide a therapeutic benefit, they first should arrive and then be absorbed at the lungs.

Besides the formoterol fumarate dihydrate and budesonide particles, the dry powder formulation according to the present invention comprises the carrier agent namely lactose monohydrate, in two, three, four or more fractions to dilute and carry the drug particles, with the carrier comprising fine carrier particles and coarse carrier particles.

According to the present invention, the fine carrier particles namely lactose monohydrate, in the dry powder formulation adhere to the surface of the coarse carrier particles namely lactose monohydrate, and prevents the drug particles from being strongly adhered on the coarse carrier particles. The fine lactose monohydrate particles used with the coarse carrier particles optimize the interaction between the coarse carrier particles and the drug particles adhered on the coarse carrier particles, and thus prevent the separation of the drug particles from the carrier particles during production, transport, and storage; and during inhalation, allow the drug particles to easily leave the carrier particles in an inhalable size to be delivered to the lungs. Thus, the facts that the dry powder formulation according to the present invention has a good flowability and can be portioned with a high dose accuracy, the dry powder formulation further contributes in that the formoterol fumarate dihydrate and budesonide particles, i.e. the active agent particles, show a good performance in their delivery to the lungs with respect to the emitted dose from the inhaler and the fine particle fraction (inhalable fraction).

According to the present invention, the term "emitted dose" refers to the portion of a total dose which is administered to the patient during inhalation from the inhaler device. The term "fine particle dose (FPD)", in turn, refers to the dose amount of the particles with an inhalable size (below 5 µm) delivered to the lungs. The term "fine particle fraction (FPF)" refers to the percentage of the fine particle dose in the total dose. In order to achieve a good performance, while dry powder formulations are developed, it is typically aimed that the emitted dose is above 90% and the FPF is above 15%.

On the other hand, the particle size of the formoterol fumarate dihydrate and the budesonide and the lactose monohydrate used in the dry powder formulation is preferably measured by means of laser diffraction method. More specifically, the particle size of the active agent is measured using a liquid dispersion method making use of water as a dispensing agent on a "Malvern Mastersizer 2000 Particle Size Analyzer". The particle size of the carrier agent, in turn, is measured using a dry dispersion method making use of air as a dispensing agent on a "Malvern Mastersizer 2000 Particle Size Analyzer". The d_{0.5} and d_{0.9} values of the active agent and the carrier agent used in the dry powder formulation according to the present invention are obtained using the measurements described above.

According to the present invention, the d_{0.5} particle size by volume defines the maximum particle size of less than 50% by volume of the particles, whereas the d_{0.9} particle size by volume defines the maximum particle size of less than 90% by volume of the particles.

The particle size of the carrier particles in the dry powder formulations administered via inhalation is among the significant factors which influence the poured bulk density and therefore the flowability of the formulation. Accordingly, the d_{0.5} particle size by volume of the fine lactose monohydrate particles is between 0.5 µm and 25 µm, preferably between 1 µm and 20 µm, whereas the d_{0.5} particle size by volume of the coarse lactose monohydrate particles is between 40 µm and 200 µm, preferably between 50 µm and 120 µm.

According to the present invention, the d_{0.9} particle size by volume of the fine lactose monohydrate particles is lower than the d_{0.5} particle size by volume of the coarse lactose monohydrate particles therein. Here, the d_{0.9} particle size by volume of the fine lactose monohydrate particles is below 60 µm, preferably between 1 µm and 50 µm, and more preferably between 5 µm and 48 µm.

Additionally, the weight ratio of the fine lactose monohydrate particles to the coarse lactose monohydrate particles is between 1:99 and 50:50, preferably between 3:97 and 45:55, and more preferably between 5:95 and 30:70 in the dry powder formulation according to the present invention.

The fine carrier particles and the coarse carrier particles are of the same agent. The carrier agent is lactose monohydrate. The active agents are formoterol and budesonide in a mixture thereof.

The d_{0.5} particle size by volume of the formoterol fumarate dihydrate and the budesonide is below 5 µm and this size corresponds to the inhalable particle size.

Within scope of the invention, when the particle sizes of the fine carrier particles and coarse carrier particle, and the ratio by weight of the fine carrier particles to the coarse carrier particles are in the ranges indicated above, the poured bulk density of the formulation is between 0.42 g/ml and 0.57 g/ml. The dry powder formulation further comprises at least one additive selected from a group comprising magnesium stearate, stearic acid, sodium lauryl sulfate, sodium stearyl fumarate, stearyl alcohol, sodium benzoate, silicon dioxide (Aerosil), amino acids (leucine, lysine, valine, methionine, and phenylalanine), or the mixtures thereof.

The dry powder formulation is formulated to comprise 1 µg to 4000 µg, preferably 2 µg to 2500 µg, more preferably 3 µg to 1000 µg of a micronized active agent per dose; 25 µg to 50 mg, preferably 50 µg to 40 mg, and more preferably 100 µg to 30 mg of a carrier agent per dose.

In order to carry out another object of the present invention, the dry powder formulation is produced using methods which are known to those skilled person in the art. In the preparation of the dry powder formulation, the active agent particles and the fine carrier particles is in a finely divided form so as to have the particle sizes given above. The components of the formulation can be produced at the respective particle sizes given above using methods such as grinding, microparticulation, and direct precipitation which are known to those skilled person in the art.

In order to prepare the dry powder formulation, the active agent, the fine carrier particles and the coarse carrier particles are mixed together at the same time or they are added sequentially and then mixed together, or first the fine carrier particles and the coarse carrier particles are mixed and then the resulting mixture is mixed with the active agent.

A method for producing the formulation according to the present invention is preferably comprising the following steps:
a. the micronized formoterol fumarate dihydrate and budesonide, the fine lactose monohydrate particles and the coarse lactose monohydrate particles are weighed,
b. the fine lactose monohydrate particles and the coarse lactose monohydrate particles are mixed for some time period (mixture A),
c. the formoterol fumarate dihydrate and budesonide are mixed with mixture A and the resulting mixture is passed through a sieve (mixture B),
d. after the mixture is mixed for some more time, the resulting final mixture is filled into suitable packages (blister cavities, capsules or reservoirs).

After all formoterol fumarate dihydrate and budesonide and the lactose monohydrate particles are mixed in order to contribute to a uniform mixing of the powder particles, these components are passed through a sieve with a mesh size between 0.10 mm and 0.45 mm, and then subjected to another mixing step for 50 to 100 minutes. Thus, the resulting mixture is homogenized without causing any change in the particle size thereof.

Preferably, an "electrostatic eliminator" is used throughout the process to reduce the electrostatic charges of the particles of a micronized size.

The dry powder formulations is used in the treatment of respiratory diseases selected from asthma and chronic obstructive pulmonary disease and other obstructive airways diseases.

The formulations are particularly used in the treatment, prevention, or in the alleviation of the symptoms of respiratory diseases comprising asthma, acute respiratory distress syndrome, chronic pulmonary inflammatory disease, bronchitis, chronic bronchitis, chronic obstructive pulmonary disease and silicosis, and similar diseases, as well as of immune disorders and conditions comprising allergic rhinitis and chronic sinusitis.

The dry powder formulations may be administered using any known types of dry powder delivery systems (particularly inhalers). The drug delivery system may be a single-dose, a multi-dose or a breath-operated system.

The present invention is elaborated in the following examples. These examples are not limiting the scope of the present invention and are to be considered under the light of the foregoing detailed disclosure.

### Example 1 (comparative out of the scope)

0.7434 mg fine lactose monohydrate having a d_{0.9} particle size by volume of about 42 µm and 24.0366 mg coarse lactose monohydrate having a d_{0.5} particle size by volume of about 58 µm as determined by laser diffraction method (Malvern analysis) are mixed in a Turbula mixer until a homogeneous mixture is obtained (mixture A). The resulting mixture A is mixed with 0.2200 mg of micronized budesonide having a d_{0.5} particle size by volume of about 2 µm (mixture B). The mixture B is passed through a 0.25 mm-sieve without causing any change in the particle size. The resulting mixture is mixed in the Turbula mixer for 75 minutes more to give a homogeneous mixture. Meanwhile, an electrostatic eliminator is used throughout the production process to reduce the electrostatic charges of the substances having a micronized particle size in the formulation. The poured bulk density of the final mixture is 0.51 g/ml as measured using some known methods as described in "Powder testing guide: Methods of measuring the physical properties of Bulk powders," L.Svarovsky, Elsevier Applied Science 1987, pp. 84-86.
The powder mixture for the dry powder inhaler prepared as described above is filled into capsules.

### Example 2 (comparative out of the scope)

0.7368 mg finer lactose monohydrate having a d_{0.9} particle size by volume of about 42 µm and 23.8232 mg coarse lactose monohydrate having a d_{0.5} particle size by volume of about 58 µm as determined by laser diffraction method (Malvern analysis) are mixed in a Turbula mixer until a homogeneous mixture is obtained (mixture A). The resulting mixture A is mixed with 0.4400 mg of micronized budesonide having a d_{0.5} particle size by volume of about 2 µm (mixture B). The mixture B is passed through a 0.25 mm-sieve without causing any change in the particle size. The resulting mixture is mixed in the Turbula mixer for 75 minutes more to give a homogeneous mixture. Meanwhile, an electrostatic eliminator is used throughout the production process to reduce the electrostatic charges of the substances having a micronized particle size in the formulation. The poured bulk density of the final mixture is 0.51 g/ml as measured using some known methods as described in "Powder testing guide: Methods of measuring the physical properties of Bulk powders," L.Svarovsky, Elsevier Applied Science 1987, pp. 84-86.
The powder mixture for the dry powder inhaler prepared as described above is filled into capsules.

### Example 3 (comparative out of the scope)

1.2494 mg fine lactose monohydrate having a d_{0.9} particle size by volume of about 10 µm and 23.7384 mg coarse lactose monohydrate having a d_{0.5} particle size by volume of about 65 µm as determined by laser diffraction method (Malvern analysis) are mixed in a Turbula mixer until a homogeneous mixture is obtained (mixture A). The resulting mixture A is mixed with 0.01224 mg of micronized formoterol fumarate dihydrate having a d_{0.5} particle size by volume of about 2 µm (mixture B). The mixture B is passed through a 0.25 mm-sieve without causing any change in the particle size. The resulting mixture is mixed in the Turbula mixer for 75 minutes more to give a homogeneous mixture. Meanwhile, an electrostatic eliminator is used throughout the production process to reduce the electrostatic charges of the substances having a micronized particle size in the formulation. The poured bulk density of the final mixture is 0.45 g/ml as measured using some known methods as described in "Powder testing guide: Methods of measuring the physical properties of Bulk powders," L.Svarovsky, Elsevier Applied Science 1987, pp. 84-86.
The powder mixture for the dry powder inhaler prepared as described above is filled into capsules.

### Example 4 (comparative out of the scope)

0.1474 mg fine lactose monohydrate having a d_{0.9} particle size by volume of about 42 µm and 4.7646 mg coarse lactose monohydrate having a d_{0.5} particle size by volume of about 58 µm as determined by laser diffraction method (Malvern analysis) are mixed in a Turbula mixer until a homogeneous mixture is obtained (mixture A). The resulting mixture A is mixed with 0.2200 mg of micronized budesonide having a d_{0.5} value by volume of about 2 µm (mixture B). The mixture B is passed through a 0.25 mm-sieve without causing any change in the particle size. The resulting mixture is mixed in the Turbula mixer for 75 minutes more to give a homogeneous mixture. Meanwhile, an electrostatic eliminator is used throughout the production process to reduce the electrostatic charges of the substances having a micronized particle size in the formulation. The poured bulk density of the final mixture is 0.51 g/ml as measured using some known methods as described in "Powder testing guide: Methods of measuring the physical properties of Bulk powders," L.Svarovsky, Elsevier Applied Science 1987, pp. 84-86.
The powder mixture for the dry powder inhaler prepared as described above is filled into blister cavities.

### Example 5 (comparative out of the scope)

0.2499 mg fine lactose monohydrate having a d_{0.9} particle size by volume of about 10 µm and 4.7477 mg coarse lactose monohydrate having a d_{0.5} particle size by volume of about 65 µm as determined by laser diffraction method (Malvern analysis) are mixed in a Turbula mixer until a homogeneous mixture is obtained (mixture A). The resulting mixture A is mixed with 0.01224 mg of micronized formoterol fumarate dihydrate having a d_{0.5} particle size by volume of about 2 µm (mixture B). The mixture B is passed through a 0.25 mm-sieve without causing any change in the particle size. The resulting mixture is mixed in the Turbula mixer for 75 minutes more to give a homogeneous mixture. Meanwhile, an electrostatic eliminator is used throughout the production process to reduce the electrostatic charges of the substances having a micronized particle size in the formulation. The poured bulk density of the final mixture is 0.45 g/ml as measured using some known methods as described in "Powder testing guide: Methods of measuring the physical properties of Bulk powders," L.Svarovsky, Elsevier Applied Science 1987, pp. 84-86.
The powder mixture for the dry powder inhaler prepared as described above is filled into blister cavities.

### Example 6

1.6592 mg fine lactose monohydrate having a d_{0.9} particle size by volume of about 25 µm and 23.3188 mg coarse lactose monohydrate having a d_{0.5} particle size by volume of about 75 µm as determined by laser diffraction method (Malvern analysis) are mixed in a Turbula mixer until a homogeneous mixture is obtained (mixture A). The resulting mixture A is mixed with a combination of 0.2200 mg of micronized budesonide and 0.01224 mg of micronized formoterol fumarate dihydrate having a d_{0.5} particle size by volume of about 2 µm (mixture B). The mixture B is passed through a 0.3 mm-sieve without causing any change in the particle size. The resulting mixture is mixed in the Turbula mixer for 75 minutes more to give a homogeneous mixture. Meanwhile, an electrostatic eliminator is used throughout the production process to reduce the electrostatic charges of the substances having a micronized particle size in the formulation. The poured bulk density of the final mixture is 0.49 g/ml as measured using some known methods as described in "Powder testing guide: Methods of measuring the physical properties of Bulk powders," L.Svarovsky, Elsevier Applied Science 1987, pp. 84-86.
The powder mixture for the dry powder inhaler prepared as described above is filled into capsules.

### Example 7

0.3291 mg fine lactose monohydrate having a d_{0.9} particle size by volume of about 25 µm and 4.6249 mg coarse lactose monohydrate having a d_{0.5} particle size by volume of about 75 µm as determined by laser diffraction method (Malvern analysis) are mixed in a Turbula mixer until a homogeneous mixture is obtained (mixture A). The resulting mixture A is mixed with a combination of 0.2200 mg of micronized budesonide and 0.01224 mg of micronized formoterol fumarate dihydrate having a d_{0.5} particle size by volume of about 2 µm (mixture B). The mixture B is passed through a 0.3 mm-sieve without causing any change in the particle size. The resulting mixture is mixed in the Turbula mixer for 75 minutes more to give a homogeneous mixture. Meanwhile, an electrostatic eliminator is used throughout the production process to reduce the electrostatic charges of the substances having a micronized particle size in the formulation. The poured bulk density of the final mixture is 0.49 g/ml as measured using some known methods as described in "Powder testing guide: Methods of measuring the physical properties of Bulk powders," L.Svarovsky, Elsevier Applied Science 1987, pp. 84-86.

The powder mixture for the inhaler dry powder prepared as described above is filled into blister cavities.

## Claims

1. A dry powder formulation comprising
a. a micronized formoterol fumarate dihydrate and micronized budesonide and,
b. lactose monohydrate as a carrier comprising fine lactose monohydrate particles and coarse lactose monohydrate particles; d_{0.5} particle size by volume of the fine lactose monohydrate particles is between 0.5 µm and 25 µm and d_{0.5} particle size by volume of the coarse lactose monohydrate particles is between 40 µm and 200 µm
**characterized in that** the poured bulk density of the dry powder formulation is between 0.42 g/ml and 0.57 g/ml.

2. The dry powder formulation according to claim 1, wherein the d_{0.9} particle size by volume of the fine lactose monohydrate particles is lower than the d_{0.5} particle size by volume of the coarse lactose monohydrate particles.

3. The dry powder formulation according to claim 2, wherein the d_{0.9} particle size by volume of the fine lactose monohydrate particles is below 60 µm.

4. The dry powder formulation according to any of the preceding claims, wherein the weight ratio of the fine lactose monohydrate particles to the coarse lactose monohydrate particles is between 1:99 and 50:50.

5. The dry powder formulation according to any of the preceding claims, wherein the formulation comprises 1 µg to 4000 µg of micronized formoterol fumarate dihydrate and micronized budesonide and 25 µg to 50 mg of lactose monohydrate per dose.

## Patentansprüche

1. Eine Trockenlöschmittelzusammensetzung, umfassend
a. ein mikronisiertes Formoterol-Fumarat-Dihydrat und mikronisiertes Budesonid und,
b. Laktose-Monohydrat als Träger, umfassend feinkörnige Laktose-Monohydrat Partikel und grobkörnige Laktose-Monohydrat-Partikel, wobei die d_{0.5} Volumen-Partikelgröße der feinkörnigen Laktose-Monohydrat-Partikel zwischen 0.5 µm und 25 µm liegt und die d_{0.5} Volumen-Partikelgröße der grobkörnigen Laktose-Monohydrat Partikel zwischen 40 µm und 200 µm liegt,
**dadurch gekennzeichnet, dass** die Schüttgutdichte der Trockenlöschmittelzusammensetzung zwischen 0,42 g/ml und 0,57 g/ml liegt.

2. Trockenlöschmittelzusammensetzung gemäß Anspruch 1, **dadurch gekennzeichnet, dass** die d_{0.9} Volumen-Partikelgröße der feinkörnigen Laktose-Monohydrat-Partikel kleiner ist als die d_{0.5} Volumen-Partikelgröße der grobkörnigen Laktose-Monohydrat-Partikel.

3. Trockenlöschmittelzusammensetzung gemäß Anspruch 2, **dadurch gekennzeichnet, dass** die d_{0.9} Volumen-Partikelgröße der feinkörnigen Laktose-Monohydrat-Partikel unter 60 µm liegt.

4. Trockenlöschmittelzusammensetzung gemäß einem der vorangegangenen Ansprüche, **dadurch gekennzeichnet, dass** das Gewichtsverhältnis der feinkörnigen Laktose-Monohydrat-Partikel zu den grobkörnigen Laktose-Monohydrat-Partikeln zwischen 1:99 und 50:50 liegt.

5. Trockenlöschmittelzusammensetzung gemäß einem der vorangegangenen Ansprüche, **dadurch gekennzeichnet, dass** die Zusammensetzung 1 µg bis 400 µg mikronisiertes Formoterol-Fumarat-Dihydrat und mikronisiertes Budesonid umfasst und 25 µg bis 50 mg Laktose-Monohydrat per Dosis umfasst.

## Revendications

1. - Formulation de poudre sèche comprenant :
a. un fumarate de formotérol dihydraté micronisé et du budésonide micronisé ; et
b. du lactose monohydraté en tant que support comprenant de fines particules de lactose monohydraté et des particules grossières de lactose monohydraté ; la dimension de particule d_{0,5} en volume des particules fines de lactose monohydraté se situant entre 0,5 µm et 25 µm et la dimension de particule d_{0,5} en volume des particules grossières de lactose monohydraté se situant entre 40 µm et 200 µm
**caractérisée par le fait que** la masse volumique en vrac au versage de la formulation de poudre sèche se situe entre 0,42 g/ml et 0,57 g/ml.

2. - Formulation de poudre sèche selon la revendication 1, dans laquelle la dimension de particule d_{0,9} en volume des fines particules de lactose monohydraté est inférieure à la dimension de particule d_{0,5} en volume des particules grossières de lactose monohydraté.

3. - Formulation de poudre sèche selon la revendication 2, dans laquelle la dimension de particule d_{0,9} en volume des fines particules de lactose monohydraté se situe au-dessous de 60 µm.

4. - Formulation de poudre sèche selon l'une quelconque des revendications précédentes, dans laquelle le rapport en poids des fines particules de lactose monohydraté aux particules grossières de lactose monohydraté se situe entre 1:99 et 50:50.

5. - Formulation de poudre sèche selon l'une quelconque des revendications précédentes, dans laquelle la formulation comprend 1 µg à 4000 µg de fumarate de formotérol dihydraté micronisé et de budésonide micronisé et 25 µg à 50 mg de lactose monohydraté par dose.
